# EUROPÄISCHE PATENTSCHRIFT

(11) **EP 0 884 022 B1**
(45) Veröffentlichungstag und Bekanntmachung des Hinweises auf die Patenterteilung: **18.02.2004**
(21) Anmeldenummer: 98106688.9
(22) Anmeldetag: 11.04.1998
(51) Int. Cl.: A61B 13/00

(54) **Zungenhalter**
Tongue depressor
Abaisse-langue

(30) Priorität: 10.06.1997 DE 29710059 U
(43) Veröffentlichungstag der Anmeldung: 16.12.1998
(73) Patentinhaber: Troost, Piet, Dr., 48346 Ostbevern (DE)
(72) Erfinder: Troost, Piet, Dr., 48346 Ostbevern (DE)
(74) Vertreter: Habbel, Hans-Georg, Dipl.-Ing.

(56) Entgegenhaltungen:
- EP-A- 0 699 421
- US-A- 1 405 689
- US-A- 2 831 480
- US-A- 3 768 477
- US-A- 5 460 524

## Beschreibung

Die Erfindung betrifft einen Zungenhalter, der von Zahnärzten verwendet werden kann.

Bei einigen Eingriffen des Zahnarztes ist es wichtig, zu verhindern, daß die Zunge in den eigentlichen Bereich der behandelten Zähne gerät. Beispielsweise ist dies notwendig, wenn Zahnabdrücke angefertigt werden, wobei vor dem Aufbringen der eigentlichen Abdruckmasse die Zähne gesäubert und speichelfrei gemacht werden, so daß die Abdruckmasse einen sicheren Halt auf den jeweiligen Zähnen hat.

Beim natürlichen Schluckreflex wird die Zunge vom Patienten unwillkürlich bewegt und gerät häufig an die zu behandelnden Zähne, so daß eine erneute, ungewünschte Befeuchtung der Zähne mit Speichel erfolgt. Dieses Problem tritt insbesondere dann auf, wenn Zähne im hinteren Gaumenbereich behandelt werden müssen.

Eine befriedigende Lösung dieses Problemes ist bislang nicht bekannt und durch das ungewollte Wiederbefeuchten der zu behandelnden Zähne mit Speichel erhöht sich der Arbeitsaufwand bzw. die Behandlungszeit des Patienten, und da häufig ein Befeuchten der zu behandelnden Zähne nicht vollständig ausgeschlossen werden kann, werden zum Teil unbefriedigende Abdruckergebnisse erhalten bzw. der Arbeitsvorgang muß wiederholt werden.

Aus der US 5,460,524 ist eine Vorrichtung bekannt, mit der die Zunge in den hinteren Mundraum geschoben und dort fixiert werden kann. Bei dieser Vorrichtung erfolgt die Fixierung dieses Zungenhalters über ein Bissstück, das zwischen den Frontzähnen des Ober- und Unterkiefers festgeklemmt wird. Diese Art der Zungenfixierung hat den Nachteil, dass, um den Zungenhalter fixieren zu müssen, es erforderlich ist, dass der Mund des Patienten so weit geschlossen wird, dass das Bissstück durch die Zähne des Ober- und Unterkiefers festgeklemmt wird, so dass die Zähne für den Zahnarzt nur schlecht erreichbar sind. Ein weiterer Nachteil dieses Zungenhalters ist, dass nicht sämtliche Zähne des Unterkiefers für den Zahnarzt erreichbar sind, um z. B. Zahnabdrücke von diesen Zähnen zu machen, da das zur Fixierung des Zungenhalters erforderliche Bissstück auf einem bzw. mehreren Zähnen des Unterkiefers aufliegt.

Der Erfindung liegt die Aufgabe zugrunde, einen Zungenhalter derart auszubilden, dass er es zum einen ermöglicht, die Zunge derart zu fixieren, dass sämtliche Zähne des Unterkiefers nicht von der Zunge erfasst werden und es andererseits ermöglicht, dass sämtliche Zähne des Unterkiefers für den behandelnden Arzt zugänglich sind. Vorteilhafte Ausgestaltungen der Erfindung sind in den Unteransprüchen erläutert.

Ein Ausführungsbeispiel der Erfindung wird nachfolgend in den Zeichnungen dargestellt, wobei
- Fig. 1: eine seitliche Gesamtansicht des vorgeschlagenen Zungenhalters darstellt,
- Fig. 2: zeigt eine Ansicht des Zungenhalters von oben,
- Fig. 3: stellt eine Ansicht von vorne dar und
- Fig. 4: zeigt eine Ansicht eines gerade verwendeten, im Mund befindlichen Zungenhalters.

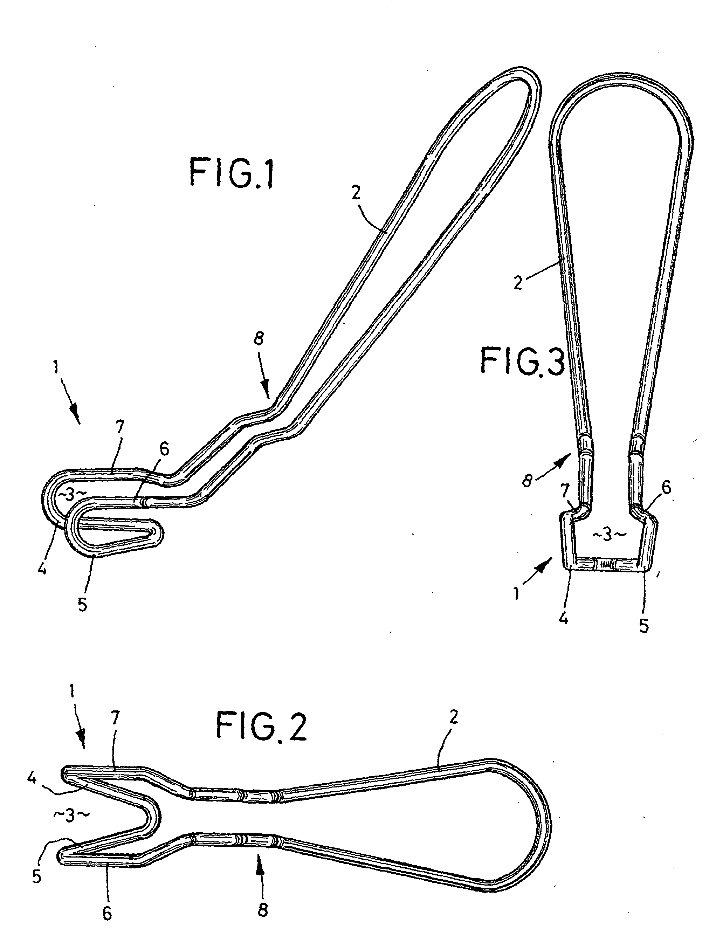

Bezugnehmend auf die Fig. 1 bis 3 ist ein Zungenhalter 1 dargestellt, der im wesentlichen aus einem Handgriff 2 und einer Aufnahmetasche 3 für die zu fixierende Zunge besteht. Der dargestellte Zungenhalter 1 ist aus einem Draht mit rundem Querschnitt hergestellt und ist daher sehr leicht und einfach zu handhaben. Selbstverständlich kann der Zungenhalter 1 auch aus einem anderen Material bzw. in einer anderen Form hergestellt werden. Der Draht ist in vorteilhafter Ausgestaltung aus rostfreiem Edelstahl hergestellt.

Die Aufnahmetasche 3 ist an ihrem vorderen, d. h. dem Handgriff 2 abgewandten Ende offen ausgebildet, um die zu fixierende Zunge zumindest teilweise aufnehmen zu können. In ihrem hinteren Bereich ist die Aufnahmetasche geschlossen ausgebildet, um eine einmal aufgenommene Zunge sicher in Richtung des Rachenraumes schieben zu können, um zu erreichen, daß die derart nach hinten verschobene Zunge die zu bearbeitenden Zähne freigibt.

Wie insbesondere aus den Fig. 2 und 3 ersichtlich wird, besteht die Aufnahmetasche aus unteren Seitenhalterungen 4 und 5 sowie oberen Seitenhalterungen 6 und 7, um die Zunge seitlich einerseits sicher zu halten und andererseits, um Schmerzen durch diese großflächige Fixierung der Zungenseitenränder zu verhindern. Es ist selbstverständlich auch möglich, flächige Seitenhalterungen zu verwenden, wobei der dargestellte Zungenhalter 1 aus gebogenem Draht den Vorteil hat, daß er zum einen günstig herzustellen und zum anderen aufgrund seines geringen Gewichtes leicht handhabbar ist und zudem aufgrund seiner geringen Größe in einfacher Weise zu desinfizieren ist.

Die Aufnahmetasche 3 ist in diesem Ausführungsbeispiel konisch ausgebildet, d. h. die Aufnahmetasche 3 weitet sich zu ihrer vorderen Aufnahmeöffnung hin, um die zu fixierende Zunge sicher aufnehmen zu können, die ebenfalls in Richtung des Rachens breiter wird. Zudem wird durch diese Ausformung erreicht, daß im vorderen Mundbereich dem Zahnarzt möglichst wenig Raum durch den Zungenhalter genommen wird, so daß er in einfacher Weise die zu bearbeitenden Zähne mit anderen Hilfsmitteln erreichen kann.

Darüber hinaus weitet sich die Aufnahmetasche 3 nach oben hin, um auch durch diese Ausformung der Anatomie der zu fixierenden Zunge gerecht zu werden und um im unteren Bereich einen möglichst geringen Platzbedarf zu benötigen, um auch hier ein einfaches Erreichen der zu bearbeitenden Zähne durch den Zahnarzt zu ermöglichen.

Der Handgriff 2 ist in vorteilhafter Ausgestaltung derart ausgebildet, daß er nach oben weist, um dort in einfacher Weise von dem Zahnarzt bzw. einer helfenden Person gehalten werden zu können.

Der Handgriff 2 weist in einem Bereich 8 eine Verflachung auf, durch die eine bessere Handhabung des Zungenhalters ermöglicht wird, da durch diesen Richtungswechsel des Handgriffes 2, der den Handgriff 2 im wesentlichen von der Aufnahmetasche 3 wegführt, so daß der obere Teil des Handgriffes 2 nicht mit der Nase bzw. den Schneidezähnen des Patienten kollidiert.

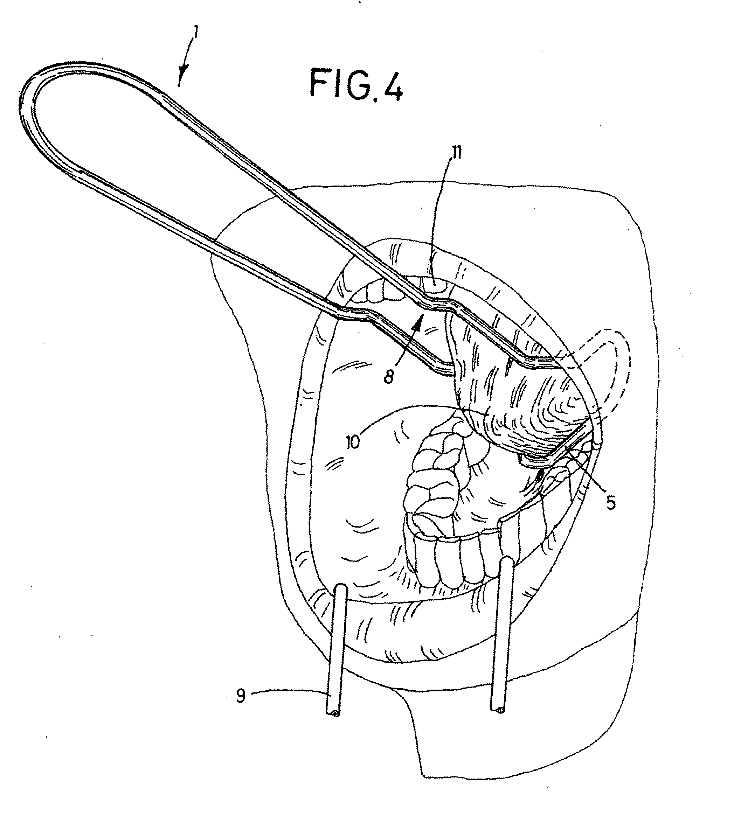

Bezugnehmend auf Fig. 4 wird der Zungenhalter 1 in seiner Gebrauchsstellung gezeigt, wobei in dieser Abbildung eine weitere Gerätschaft zu sehen ist, nämlich ein Haltebügel 9, mit der die Unterlippe des Patienten fixiert werden kann. In dieser Fig. 4 ist ersichtlich, daß die Zunge 10 durch den Zungenhalter 1 sowohl fixiert wird als auch in dieser fixierten Stellung nach hinten in Richtung des Rachenraumes geschoben werden kann, um den jeweils zu behandelnden Zahnraum bzw. sämtliche Zähne, beispielsweise des Unterkiefers, von der Zunge 10 freizubekommen.

Durch die Verflachung bzw. den flacheren Bereich 8 wird erreicht, daß der Zungenhalter 1 nicht mit den vorderen Schneidezähnen 11 des Patienten kollidiert bzw. mit der Nase des Patienten.

## Patentansprüche

1. Zungenhalter, mit einer Aufnahmetasche für die zu haltende Zunge, deren vorderes, der Zunge zugewandtes Ende offen ausgebildet ist und deren Innenraum von Seitenhalterungen seitlich begrenzt ist, **gekennzeichnet durch** einen an der Aufnahmetasche (3) angeordneten Handgriff (2), der in der Gebrauchshaltung des Zungenhalters (1) nach oben verlaufend vom Unterkiefer sich entfernend ausgebildet ist und sich bis außerhalb der Mundhöhle erstreckt.

2. Zungenhalter gemäß Anspruch 1, **gekennzeichnet durch** die Aufnahmetasche (3), die in ihrem hinteren, dem Handgriff (2) zugewandten Ende geschlossen ausgebildet ist.

3. Zungenhalter gemäß Anspruch 1 oder 2, **gekennzeichnet durch** die Aufnahmetasche (3), deren Innenraum am vorderen Ende weiter ausgebildet ist als ihr hinteres Ende.

4. Zungenhalter gemäß Anspruch 3, **gekennzeichnet durch** die Seitenhalterungen (4, 5, 6, 7), die zum griffseitigen Ende der Aufnahmetasche (3) konisch aufeinander zulaufend ausgebildet sind.

5. Zungenhalter gemäß einem der vorhergehenden Ansprüche, **gekennzeichnet durch** den Handgriff (2), der in seiner Gebrauchshaltung einen Bereich (8) aufweist, indem er eine geringere Steigung aufweist als im übrigen Bereich des Handgriffes (2).

6. Zungenhalter gemäß einem der vorhergehenden Ansprüche, **gekennzeichnet durch** die aus Draht hergestellten Bestandteile.

7. Zungenhalter gemäß einem der vorhergehenden Ansprüche, **gekennzeichnet durch** den einteilig ausgebildeten Zungenhalter (1).

8. Zungenhalter gemäß einem der vorhergehenden Ansprüche, **gekennzeichnet durch** den aus Metall gefertigten Zungenhalter (1).

9. Zungenhalter gemäß einem der vorhergehenden Ansprüche, **gekennzeichnet durch** den unteren Bereich der Aufnahmetasche (3), der enger ausgebildet ist als der obere Bereich der Aufnahmetasche (3).

## Claims

1. Tongue depressor with a receiving pocket for the tongue to be depressed, the front end of which that is turned towards the tongue, is designed to be open and the interior of which is limited at the side by lateral supports, **characterised by** a hand grip (2) arranged on the receiving pocket (3) which is designed running upwards away from the lower jaw in the position of use of the tongue depressor (1) and extends outside the mouth cavity.

2. Tongue depressor according to claim 1, **characterised by** the receiving pocket (3), which is designed closed at the rear end, which is turned towards the hand grip (2).

3. Tongue depressor according to claim 1 or 2, **characterised by** the receiving pocket (3), the inside area of which is designed wider at the front end than at the rear end.

4. Tongue depressor according to claim 3, **characterised by** lateral supports (4, 5, 6, 7), which are designed running conically to each other on the grip end of the receiving pocket (3).

5. Tongue depressor according to one of the previous claims, **characterised by** the hand grip (2), which in its position of use has an area (8), in which it has a smaller gradient than in the remaining area of the hand grip (2).

6. Tongue depressor according to one of the previous claims, **characterised by** components made out of wire.

7. Tongue depressor according to one of the previous claims, **characterised by** the tongue depressor (1) designed in one piece.

8. Tongue depressor according to one of the previous claims, **characterised by** the tongue depressor (1) made of metal.

9. Tongue depressor according to one of the previous claims, **characterised by** the lower area of the receiving pocket (3), which is made narrower than the upper area of the receiving pocket (3).

## Revendications

1. Repousse-langue avec une poche de retenue pour la langue à retenir, dont l'extrémité antérieure orientée vers la langue est ouverte et dont l'espace intérieur est délimité latéralement par des fixations latérales, **caractérisé en ce qu'**il comporte une poignée (2) disposée sur la poche de retenue (3), qui s'éloigne de la mâchoire inférieure vers le haut dans la position d'utilisation du repousse-langue (1) et s'étend jusqu'à l'extérieur de la cavité buccale.

2. Repousse-langue selon la revendication 1, **caractérisé en ce que** la poche de retenue (3) est fermée à son extrémité postérieure orientée vers la poignée (2).

3. Repousse-langue selon la revendication 1 ou 2, **caractérisé en ce que** la poche de retenue (3) a un espace intérieur plus large à l'extrémité antérieure que son extrémité postérieure.

4. Repousse-langue selon la revendication 3, **caractérisé en ce que** les fixations latérales (4, 5, 6, 7) convergent les unes vers les autres en forme de cône vers l'extrémité de la poche de retenue (3) située du côté de la poignée.

5. Repousse-langue selon l'une ou l'ensemble des revendications précédentes, **caractérisé en ce que** la poignée (2) présente dans sa position d'utilisation une zone (8) où son inclinaison est plus faible que dans le reste de la poignée (2).

6. Repousse-langue selon l'une ou l'ensemble des revendications précédentes, **caractérisé en ce que** les éléments sont fabriqués en fil métallique.

7. Repousse-langue selon l'une ou l'ensemble des revendications précédentes, **caractérisé en ce que** le repousse-langue (1) est construit d'une pièce.

8. Repousse-langue selon l'une ou l'ensemble des revendications précédentes, **caractérisé en ce que** le repousse-langue (1) est fabriqué en métal.

9. Repousse-langue selon l'une ou l'ensemble des revendications précédentes, **caractérisé en ce que** la partie inférieure de la poche de retenue (3) est plus étroite que la partie supérieure de la poche de retenue (3).
